# EUROPEAN PATENT APPLICATION

(11) **EP 4 063 355 A1**
(43) Date of publication of application: **28.09.2022**
(21) Application number: 21163952.1
(22) Date of filing: 22.03.2021
(51) Int. Cl.: C07D 301/12, C07C 5/32, C07C 15/46, B01J 19/00

(54) **INTEGRATED PROCESS AND PLANT FOR MAKING STYRENE AND PROPENE OXIDE**

(71) Applicant: Evonik Operations GmbH, 45128 Essen (DE); thyssenkrupp Industrial Solutions AG, 45143 Essen (DE)
(72) Inventor: BOLZ, David, 60594 Frankfurt (DE); WIEDERHOLD, Holger, 64297 Darmstadt (DE)
(74) Representative: Evonik Patent Association

(57) **Abstract**

An integrated process for making styrene and propene oxide which comprises the steps:
a) dehydrogenating ethylbenzene in the presence of a dehydrogenation catalyst;
b) separating styrene and hydrogen from the reaction mixture of step a);
c) producing hydrogen peroxide from hydrogen separated in step b) and oxygen;
d) reacting propene with the hydrogen peroxide obtained in step c) in the presence of an epoxidation catalyst to provide a reaction mixture comprising propene oxide; and
e) separating propene oxide from the reaction mixture obtained in step d).

## Description

### Field of the invention

The invention is directed at an integrated process and an integrated plant for making styrene and propene oxide where the ratio of styrene production to propene oxide production can be varied.

### Background of the invention

A coupled production of styrene and propene oxide is known from the prior art using the so-called PO-SM process where ethylbenzene is oxidized with air to ethylbenzene hydroperoxide which is then reacted with propene in the presence of an epoxidation catalyst to give 1-phenylethanol and propene oxide. The 1-phenylethanol is then dehydrated to styrene. Since the epoxidation reaction of the PO-SM process produces equimolar amounts of 1-phenylethanol and propene oxide, the production rates of styrene production and of propene oxide are inevitably coupled and the ratio of styrene production to propene oxide production cannot be varied. This is a severe disadvantage when the market demands for styrene and for propene oxide are different because the production rates then cannot be matched with the market demand.

The stand-alone production of styrene by dehydrogenation of ethylbenzene is well known from the prior art and several processes have been commercialized. An overview is given in Ullmann's Encyclopedia of Industrial Chemistry, online edition, Vol. 34, pages 529-544, DOI 10.1002/14356007.a25_329.pub2, in particular on pages 532-534. A stand-alone production of propene oxide is possible by a the so-called HPPO process where propene is epoxidized with hydrogen peroxide in the presence of a titanium zeolite catalyst. An overview of the HPPO process is given in Ullmann's Encyclopedia of Industrial Chemistry, online edition, entry Propylene Oxide, DOI 10.1002/14356007.a22_239.pub3 on pages 16-18.

### Summary of the invention

The inventors of the present invention have found a way of integrating the processes of making styrene by ethylbenzene dehydrogenation and of making propene oxide by the HPPO process which reduces raw material consumption compared to the stand-alone processes and allows for varying the ratio of styrene production to propene oxide production in the integrated process. Such integration can be provided by separating the hydrogen provided by ethylbenzene dehydrogenation, producing hydrogen peroxide from the separated hydrogen in an anthraquinone process and using the hydrogen peroxide produced by the anthraquinone process as the oxidant in the HPPO process. In contrast to the PO-SM process, the integrated process of the invention can also be implemented in an existing stand-alone plant for styrene production with little or no change to the existing styrene production equipment. The use of an additional facility for producing hydrogen allows for varying the ratio of styrene production to propene oxide production to a ratio lower than possible for a PO-SM process.

Subject of the invention is therefore an integrated process for making styrene and propene oxide which comprises the steps
a) dehydrogenating ethylbenzene in the presence of a dehydrogenation catalyst;
b) separating styrene and hydrogen from the reaction mixture of step a);
c) producing hydrogen peroxide from hydrogen separated in step b) and oxygen;
d) reacting propene with the hydrogen peroxide obtained in step c) in the presence of an epoxidation catalyst to provide a reaction mixture comprising propene oxide; and
e) separating propene oxide from the reaction mixture obtained in step d).

A further subject of the invention is an integrated plant for making styrene and propene oxide which comprises
a) an ethylbenzene dehydrogenator comprising a dehydrogenation catalyst;
b) a first work-up facility for separating styrene and hydrogen connected to an outlet for reaction mixture of the ethylbenzene dehydrogenator;
c) a hydrogen peroxide production facility with a hydrogen inlet connected to a hydrogen outlet of the first work-up facility;
d) a propene epoxidizer connected to a hydrogen peroxide outlet of the hydrogen peroxide production facility and comprising an epoxidation catalyst; and
e) a second work-up facility for separating propene oxide from an epoxidation reaction mixture connected to an outlet for reaction mixture of the propene epoxidizer.

### Brief description of drawings

The figure shows a preferred embodiment of the integrated plant of the invention comprising a hydrogen peroxide production facility with a hydrogenator, an oxidizer and an extraction column; a propene epoxidizer with a tube bundle fixed bed reactor; and heat integration between the ethylbenzene dehydrogenator and a distillation column for solvent recovery in the work-up facility for separating propene oxide.

### Detailed description of the invention

The integrated process of the invention for making styrene and propene oxide comprises the steps of
a) dehydrogenating ethylbenzene in the presence of a dehydrogenation catalyst;
b) separating styrene and hydrogen from the reaction mixture of step a);
c) producing hydrogen peroxide from hydrogen separated in step b) and oxygen;
d) reacting propene with the hydrogen peroxide obtained in step c) in the presence of an epoxidation catalyst to provide a reaction mixture comprising propene oxide; and
e) separating propene oxide from the reaction mixture obtained in step d).

In step a) of the process of the invention, ethylbenzene is dehydrogenated in the presence of a dehydrogenation catalyst. The dehydrogenation catalyst preferably comprises iron oxide as the active component and preferably further comprises chromium oxide and potassium carbonate as propotors. Suitable dehydrogenation catalysts are commercially available, for example from Clariant under the trade name StyroMax^{®} UL3. The dehydrogenation of ethylbenzene is preferably carried out in the presence of steam to prevent formation of carbon deposits on the catalyst. The dehydrogenation is typically carried out at a temperature of from 600 to 640 °C and preferably at a pressure of less than 1 bar. The dehydrogenation of ethylbenzene is an endothermal reaction and can be carried out either adiabatically or isothermally. Adiabatic ethylbenzene dehydrogenation is preferably carried out by combining ethylbenzene vapor with superheated steam and passing the mixture over a fixed bed of the dehydrogenation catalyst. Suitable reaction conditions and reactors for dehydrogenating ethylbenzene are known from the prior art and are summarized in Ullmann's Encyclopedia of Industrial Chemistry Vol. 34, pages 532-534, DOI 10.1002/14356007.a25_329.pub2.

The dehydrogenating of ethylbenzene in step a provides a reaction mixture comprising styrene and hydrogen. The reaction mixture will typically also contain nonreacted ethylbenzene. If ethylbenzene is dehydrogenated in the presence of steam, the reaction mixture will also comprise minor amounts of carbon dioxide and carbon monoxide.

In step b) of the process of the invention, styrene and hydrogen are separated from the reaction mixture of step a). The separation preferably comprises cooling the reaction mixture to condense styrene, ethylbenzene and water if steam is used in the dehydrogenation. If water is present, the condensate will typically comprise two liquid phases, an aqueous phase and an organic phase comprising styrene, nonreacted ethylbenzene and organic by-products, typically benzene and toluene. The organic phase obtained by condensation is preferably separated by a series of distillations, preferably a first distillation separating low-boiling products as an overhead product, a second distillation, separating nonreacted ethylbenzene as an overhead product from the bottoms product of the first distillation and a third distillation separating styrene from the bottoms product of the second distillation. A polymerization inhibitor is preferably added prior to the first distillation. Step b) preferably comprises separating nonreacted ethylbenzene from the reaction mixture of step a) and recycling the separated ethylbenzene to step a).

The hydrogen formed in the dehydrogenation is separated with the gas phase remaining after cooling and condensation, which typically contains more than # % by volume of hydrogen. If ethylbenzene is dehydrogenated in the presence of steam, the gas phase will also contain water vapor as well as some carbon dioxide and carbon monoxide. Preferably, the separated gas phase is dried and further purified by pressure swing adsorption to provide a purified hydrogen gas. Suitable methods for purifying hydrogen by pressure swing adsorption are known from the prior art. If step a) is carried out in the presence of steam and step c) uses a palladium metal catalyst for producing hydrogen peroxide, step b) preferably comprises removal of carbon monoxide from separated hydrogen which is preferably achieved by a pressure swing adsorption. Carbon monoxide is then preferably removed to a level of less than # ppm by volume. The separated gas phase may also be subjected to one or more membrane separation steps for removing by-products from hydrogen, preferably between a step of drying the separated gas phase and a step of pressure swing adsorption.

In step c) of the process of the invention, hydrogen peroxide is produced from hydrogen separated in step b) and oxygen. Additional hydrogen may be fed to step c) in order to decouple the production capacity for propene oxide in steps d) and e) from the production capacity for styrene in steps a) and b). The integrated process may therefore comprise an additional step of generating hydrogen which is supplied to step c). The additional step of generating hydrogen may be by electrolysis of water or by steam reforming.

The hydrogen separated in step b) can be reacted with oxygen in a liquid reaction medium in the presence of a noble metal catalyst in what is known as a hydrogen peroxide direct synthesis. The noble metal catalyst is preferably a supported catalyst, with alumina, silica, titanium dioxide, zirconium dioxide, zeolites and acticated carbons being preferred supports. The noble metal catalyst may be a suspended catalyst or preferably a fixed bed catalyst. The noble metal catalyst preferably comprises palladium as noble metal, optionally in combination with platinum, gold or silver, a combination of palladium with platinum at a weight ratio of Pd:Pt of more than 4 being most preferred. Oxygen can be used as pure oxygen, air or air enriched in oxygen. Direct synthesis is preferably carried out with a gas composition that is not flammable. For this purpose, an inert gas such as nitrogen or carbon dioxide can be added. Direct synthesis is preferably carried out with a gas mixture containing at most 6 % by volume hydrogen and most preferably from 3 to 5 % by volume hydrogen. The gas mixture preferably contains preferably from 10 to 50 % by volume oxygen and most preferably from 15 to 45 % by volume oxygen. Hydrogen and oxygen are preferably dispersed separately in the liquid reaction medium and inert gas can be added either to the hydrogen or to the oxygen feed. The liquid reaction medium may be an aqueous, aqueous-organic or organic reaction medium and preferably consists essentially of an alcohol or a mixture of an alcohol and water, the alcohol most preferably being methanol. The liquid reaction medium preferably comprises a halide, more preferably iodide or bromide and most preferably bromide in an amount of 10⁻⁶ to 10⁻² mol/l, preferably 10⁻⁵ to 10⁻³ mol/I and most preferably 10⁻⁵ to 510⁻⁴ mol/I in order to suppress decomposition of hydrogen peroxide on the noble metal catalyst. The liquid reaction medium preferably further comprises a strong acid having a pKₐ of less than 3 in an amount of 0,0001 to 0,5 mol/I and preferably 0,001 bis 0,1 mol/I in order to improve selectivity for hydrogen peroxide formation, with sulfuric acid, phosphoric acid, nitric acid and methane sulfonic acid being preferred. The hydrogen peroxide direct synthesis is preferably carried out in a fixed bed reactor operated as bubble column with hydrogen, oxygen and optionally inert gas being dispersed below a catalyst fixed bed.

In a preferred embodiment, the hydrogen separated in step b) is reacted with oxygen in an anthraquinone process, preferably providing a 20 to 75 % by weight aqueous solution of hydrogen peroxide. The anthraquinone process uses a working solution comprising at least one alkylanthraquinone, alkyltetrahydroanthraquinone or a mixture of both, referred to as quinones in the following. The working solution also comprises a solvent and typically comprises at least one solvent for dissolving the quinone and at least one solvent for dissolving the hydroquinone. The alkylanthraquinone is preferably a 2-alkylanthraquinone, more preferably 2-ethylanthraquinone (EAQ), 2-amylanthraquinone (AAQ) or 2-(4-methylpentyl)-anthraquinone IHAQ and most preferably a mixture of EAQ with AAQ and/or IHAQ where the molar fraction of quinones carrying an ethyl group is from 0.05 to 0.95. The working solution preferably further comprises the corresponding 2-alkyltetrahydroanthraquinones and the ratio of 2-alkyltetrahydroanthraquinones plus 2-alkyltetrahydroanthrahydroquinones to 2-alkylanthraquinones plus 2-alkylanthrahydroquinones is preferably maintained in the range of from 1 to 20 by adjusting the conditions of the hydrogenating and regenerating steps used in the anthraquinone process. The working solution preferably comprises a mixture of alkylbenzenes having 9 or 10 carbon atoms as solvent for anthraquinones and at least one polar solvent selected from diisobutylcarbinol (DiBC), methylcyclohexylacetate (MCA), trioctylphosphate (TOP), tetrabutylurea (TBU) and N-octylcaprolactam as solvent for anthrahydroquinones, DiBC, MCA and TOP being preferred and TOP being most preferred.

The anthraquinone process is a cyclic process, comprising a hydrogenation step, where hydrogen is reacted with the working solution in the presence of a hydrogenation catalyst to convert at least part of the quinone to the corresponding hydroquinone, a subsequent oxidation step, where the hydrogenated working solution containing hydroquinone is reacted with oxygen to form hydrogen peroxide and quinone, and an extraction step, where hydrogen peroxide is extracted from the oxidized working solution with water to provide an aqueous solution of hydrogen peroxide. The reaction cycle is completed by returning the extracted working solution returned to the hydrogenation step.

In the hydrogenation step, the working solution is reacted with the hydrogen separated in step b) in the presence of a heterogeneous hydrogenation catalyst. During the reaction all or a part of the quinones are converted to the corresponding hydroquinones. All hydrogenation catalysts known from the prior art for the anthraquinone cyclic process can be used as catalysts in the hydrogenation stage. Noble metal catalysts containing palladium metal as the principal component are preferred. The catalysts can be used as a fixed bed catalyst or as a suspended catalyst and suspended catalysts can be either unsupported catalysts, such as palladium black, or supported catalysts, with suspended supported catalysts being preferred. SiO₂, TiO₂, Al₂O₃ and mixed oxides thereof, as well as zeolites, BaSO₄ or polysiloxanes, are can be used as support materials for fixed-bed catalysts or supported suspended catalysts, with TiO₂ and SiO₂/TiO₂ mixed oxides being preferred. Catalysts in the form of monolithic or honeycombed moldings, the surface of which is coated with the noble metal, can also be used. Hydrogenation can be carried out in stirred-tank reactors, tube reactors, fixed-bed reactors, loop reactors or air-lift reactors which can be equipped with devices for distributing hydrogen in the working solution, such as static mixers or injection nozzles. Preferably, a tube reactor with a recycle and a Venturi nozzle for injecting stream hydrogen into the reactor feed as known from WO 02/34668 is used. Hydrogenation is preferably carried out at a temperature of from 20 °C to 100°C, more preferably 45 to 75°C, and a pressure of from 0.1 MPa to 1 MPa, preferably 0.2 MPa to 0.5 MPa. The hydrogenation is preferably performed in such a way that essentially all hydrogen introduced into the hydrogenation reactor is consumed in a single pass through the reactor. The ratio between hydrogen and working solution fed to the hydrogenation reactor is preferably chosen to convert between 30 and 80 % of the quinones to the corresponding hydroquinones. If a mixture of 2-alkylanthraquinones and 2-alkyltetrahydroanthraquinones is used, the ratio between hydrogen and working solution is preferably chosen so that only the 2-alkyltetrahydroanthraquinones are converted to hydroquinones and the 2-alkylanthraquinones remain in the quinone form.

In the oxidation step, the hydrogenated working solution from the hydrogenation step is reacted with an oxygen-containing gas, preferably with air or with oxygen enriched air, in an oxidation reactor. All oxidation reactors known from the prior art for the anthraquinone process can be used for the oxidation, bubble columns operated in co-current being preferred. The bubble column can be free from internal devices, but preferably contains distribution devices in the form of packings or sieve plates, most preferably sieve plates in combination with internal coolers. Oxidation is preferably carried out at a temperature of from 30 to 70°C, more preferably from 40 to 60°C. Oxidation is preferably performed with an excess of oxygen to convert more than 90 %, preferably more than 95 %, of the hydroquinones to the quinone form. The oxidation step provides an oxidized working solution comprising hydrogen peroxide and an alkylanthraquinone, an alkyltetrahydroanthraquinone or both.

In the extraction step, the oxidized working solution from the oxidation step, containing dissolved hydrogen peroxide, is extracted with an aqueous solution to provide an aqueous hydrogen peroxide solution and an extracted oxidized working solution containing essentially no hydrogen peroxide. Deionized water, which may optionally contain additives for stabilizing hydrogen peroxide, adjusting the pH and/or corrosion protection, is preferably used for extracting the hydrogen peroxide. Extraction is preferably carried out in a counter-current continuous extraction column, sieve-plate columns being most preferred. The aqueous hydrogen peroxide solution obtained by extraction may be used directly in step d) for reacting with propene or may be concentrated by distilling off water at reduced pressure prior to using it in step d). The aqueous hydrogen peroxide solution obtained by extraction may also be purified, preferably by washing with a solvent, which is preferably a solvent comprised in the working solution.

The anthraquinone process preferably comprises at least one additional step for regenerating the working solution, where by-products formed in the process are converted back to quinones. Regeneration is carried out by treating hydrogenated working solution with alumina or sodium hydroxide, preferably using a side stream to the cyclic process. In addition to regeneration of hydrogenated working solution, extracted oxidized working solution may be regenerated in a side stream using alumina, sodium hydroxide or an organic amine. Suitable methods for regenerating the working solution on an anthraquinone process are known from the prior art.

In step d) of the process of the invention, propene is reacted with the hydrogen peroxide obtained in step c) in the presence of an epoxidation catalyst to provide a reaction mixture which comprises propene oxide. Propene can be used as a technical mixture with propane, preferably containing from 0.1 to 15 vol-% of propane. Both homogeneous and heterogeneous epoxidation catalysts may be used in step d). Suitable epoxidation catalysts and reaction conditions for reacting stream S6 with propene to form propene oxide are known from the prior art. Suitable homogeneous epoxidation catalysts are manganese complexes with polydentate nitrogen ligands, in particular 1,4,7-trimethyl-1,4,7-triazacyclononane ligands, as known from WO 2011/063937. Other suitable homogeneous epoxidation catalysts are heteropolytungstates and heteropolymolybdates, in particular polytungstophosphates, as known from US 5,274,140. Suitable heterogeneous epoxidation catalysts are titanium zeolites containing titanium atoms on silicon lattice positions.

Preferably, a titanium zeolite catalyst is used as the epoxidation catalyst, preferably a titanium silicalite catalyst with an MFI or MEL crystal structure, and most preferably titanium silicalite-1 with MFI structure as known from EP 0 100 119 A1, is used. The titanium zeolite catalyst is preferably employed as a shaped catalyst in the form of granules, extrudates or shaped bodies. For the forming process the catalyst may contain 1 to 99% of a binder or carrier material, all binders and carrier materials being suitable that do not react with hydrogen peroxide or with the epoxide under the reaction conditions employed for the epoxidation, silica being preferred as binder. Extrudates with a diameter of 1 to 5 mm are preferably used as fixed bed catalysts. Epoxidation with a titanium zeolite catalyst is preferably carried out in the presence of a solvent which preferably is a methanol solvent. The methanol solvent can be a technical grade methanol, a solvent stream recovered in the work-up of the epoxidation reaction mixture or a mixture of both. The epoxidation is preferably carried out at a temperature of 30 to 80°C, more preferably at 40 to 60°C, and a pressure of from 0,5 to 5 MPa, more preferably 1,5 to 3,5 MPa. The epoxidation is preferably carried out in a fixed bed reactor by passing a mixture comprising propene, hydrogen peroxide and methanol over the catalyst fixed bed. The fixed bed reactor is preferably equipped with cooling means and cooled with a liquid cooling medium. The temperature profile within this reactor is preferably maintained such that the cooling medium temperature of the cooling means is at least 40°C and the maximum temperature within the catalyst bed is 60°C at the most, preferably 55°C. The epoxidation reaction mixture is preferably passed through the catalyst bed in down flow mode, preferably with a superficial velocity from 1 to 100 m/h, more preferably 5 to 50 m/h, most preferred 5 to 30 m/h. The superficial velocity is defined as the ratio of volume flow rate/cross section of the catalyst bed. Additionally, it is preferred to pass the reaction mixture through the catalyst bed with a liquid hourly space velocity (LHSV) from 1 to 20 h⁻¹, preferably 1.3 to 15 h⁻¹. It is particularly preferred to maintain the catalyst bed in a trickle bed state during the epoxidation reaction. Suitable conditions for maintaining the trickle bed state during the epoxidation reaction are disclosed in WO 02/085873 on page 8 line 23 to page 9 line 15. Propene is preferably employed in excess relative to the hydrogen peroxide in order to achieve high hydrogen peroxide conversion, the molar ratio of propene to hydrogen peroxide preferably being chosen in the range from 1.1 to 30. The methanol solvent is preferably used in the epoxidation in a weight ratio of 0.5 to 20 relative to the amount of stream hydrogen peroxide. The amount of catalyst employed may be varied within wide limits and is preferably chosen so that a hydrogen peroxide consumption of more than 90%, preferably more than 95%, is achieved within 1 minute to 5 hours under the employed epoxidation reaction conditions. Most preferably, the epoxidation reaction is carried out with a catalyst fixed bed maintained in a trickle bed state at a pressure close to the vapor pressure of propene at the reaction temperature, using an excess of propene that provides a reaction mixture comprising two liquid phases, a methanol rich phase and a propene rich liquid phase. Two or more fixed bed reactors may be operated in parallel or in series in order to be able to operate the epoxidation process continuously when regenerating the epoxidation catalyst. Regeneration of the epoxidation catalyst can be carried out by calcination, by treatment with a heated gas, preferably an oxygen containing gas or by a solvent wash, preferably by the periodic regeneration described in WO 2005/000827.

In step e) of the process of the invention, propene oxide is separated from the reaction mixture obtained in step d).

Propene oxide may be separated from the reaction mixture by distillation or extractive distillation using methods known from the prior art. Preferably, propene oxide is separated from the reaction mixture by distillation after a pressure release stage which removes most of the non-reacted propene from the reaction mixture. Pressure release is preferably carried out in several stages with corresponding compressing stages as described in WO 2017/089079.

When a methanol solvent is used in step e) and the reaction mixture comprises methanol, the separation of propene oxide by distillation is preferably carried out in at least two columns, operating the first column to provide a crude propene oxide overhead product containing from 20 to 60 % of the methanol contained in the reaction mixture and further purifying the overhead product by at least one additional distillation. The overhead product of the first column is preferably further purified by distilling off remaining propene and propane, followed by extractive distillation, most preferably using the extractive distillation method of WO 2004/048355 for additional removal of carbonyl compounds. The bottoms product of the first column, which comprises methanol and water, is preferably separated in at least one distillation stage to provide a recovered methanol as an overhead product. For this purpose, the bottoms product of the first column may be combined with methanol separated from the crude propene oxide overhead product of the first column, such as the bottoms product of the extractive distillation as described in WO 2004/048355. The bottoms product of the first column is preferably separated in two subsequent distillation stages providing recovered methanol as an overhead product from both stages. The two distillation stages are preferably operated with a higher pressure in the second stage and overhead product vapor from the second stage is used for heating the bottoms evaporator of the first stage in order to save energy. Preferably, an acid is added to at least one of the distillation stages or prior to the distillation. When the acid is added to a distillation stage, it is preferably added at a feed point above the feed point for the solvent mixture and below the column top. The acid may also be added to the reflux stream of the distillation column. Preferably, the acid is prior to the distillation. Adding an acid in reduces the content of volatile organic amines in the recovered methanol. The acid is preferably added in an amount providing a content of less than 250 ppm by weight nitrogen in the form of organic nitrogen compounds in the recovered methanol, more preferably in an amount providing a content of less than 50 ppm by weight nitrogen in the form of organic nitrogen compounds. The acid may be a mineral acid, such as nitric acid, sulfuric acid, hydrochloric acid, phosphoric acid or perchloric acid; a sulfonic acid, such as methane sulfonic acid; or a carboxylic acid. Preferred are sulfuric acid and phosphoric acid, most preferred is sulfuric acid. The amount of nitrogen in the form of organic nitrogen compounds can be determined as the difference between the total amount of nitrogen and the amount of nitrogen in the form of inorganic nitrogen compounds. The total amount of nitrogen can be determined by the Kjeldahl method as described in DIN 53625. The recovered methanol is preferably recycled to step d) of the process of the invention.

The bottoms of the first column is preferably subjected to a catalytic hydrogenation before it is distilled for recovering methanol. When the bottoms product of the first column is combined with methanol separated from the crude propene oxide overhead product by the extractive distillation as described in WO 2004/048355, this is done prior to the catalytic hydrogenation. The catalytic hydrogenation is preferably carried out at a hydrogen partial pressure of from 0.5 to 30 MPa, more preferably of from 1 to 25 MPa and most preferably of from 1 to 5 MPa. The temperature is preferably in the range of from 80 to 180 °C, more preferably from 90 to 150 °C. The catalytic hydrogenation is carried out in the presence of a hydrogenation catalyst, preferably a heterogeneous hydrogenation catalyst. Raney nickel and Raney cobalt may be used as hydrogenation catalyst. Preferably, a supported metal catalyst comprising one or more of metals selected from the group consisting of Ru, Rh, Pd, Pt, Ag, Ir, Fe, Cu, Ni and Co on a catalyst support is used. The metal is preferably platinum, palladium, iridium, ruthenium or nickel and most preferably ruthenium or nickel. The catalyst support can be any solid which is inert and does not deteriorate under the hydrogenation conditions. Suitable as catalyst support are activated carbon, the oxides SiO₂, TiO₂, ZrO₂ and Al₂O₃, and mixed oxides comprising at least two of silicon, aluminum, titanium and zirconium. SiO₂, Al₂O₃ and mixed oxides of silicon and aluminum are preferably used as the catalyst support for the supported metal catalyst. The catalyst support is preferably shaped as spheres, pellets, tablets, granules or extrudates. Preferred are extrudates with a diameter of from 0.5 to 5mm, especially from 1 to 3 mm, and a length of from 1 to 10 mm. The supported metal catalyst preferably comprises from 0.01 to 60 wt.% metal. Supported noble metal catalysts preferably comprise from 0.1 to 5 % metal. Supported nickel and cobalt catalysts preferably comprise from 10 to 60 % metal. The supported metal catalyst may be prepared by methods known in the art, preferably by impregnating the catalyst support with a metal salt followed by reducing the metal salt to the catalytically active metal. Suitable supported metal catalysts are commercially available, for example from Clariant under the NISAT^{®} trade name and from Evonik Industries under the Octolyst^{®} trade name. The catalytic hydrogenation converts unreacted hydrogen peroxide to water and the by-product peroxides 1-hydroperoxy-2-propanol and 2-hydroperoxy-1-propanol formed in step a) to 1,2-propanediol and prevents by-product formation by peroxide decomposition in subsequent work-up stages. The catalytic hydrogenation is preferably carried out to a conversion of hydrogen peroxide that provides a hydrogenated solvent mixture containing less than 0.1 % by weight hydrogen peroxide. The hydrogenation also converts aldehyde and ketone by-products, such as acetaldehyde, to the corresponding alcohols, with the degree of conversion depending on the catalyst and the reaction conditions used. The conversion of the hydrogenation of acetaldehyde to ethanol can be adjusted by varying the reaction time and the hydrogen partial pressure and the temperature used in the catalytic hydrogenation and is preferably adjusted to provide a hydrogenated solvent mixture comprising from 1 to 1000 mg/kg of acetaldehyde.

In a preferred embodiment of the integrated process of the invention, the epoxidation catalyst in step d) is a titanium zeolite catalyst and step d) is carried out in the presence of a solvent and step e) comprises separating the solvent from the reaction mixture obtained in step d). The reaction mixture of step a) is passed through a steam generator prior to separation in step b) and steam generated in the steam generator is used in step e) to provide heat for separating the solvent. When a methanol solvent is used in step d), the separation of solvent is preferably carried out in two heat integrated distillation columns with the first distillation column operated at a higher pressure than the second distillation column, heating the bottoms evaporator of the second distillation column with steam from the steam generator and heating the bottoms evaporator of the first distillation column with overhead product vapor from the second distillation column. The heat integration between the cooling of the reaction mixture of step a) and the solvent separation in step e) reduces the overall energy consumption of the integrated process.

The integrated process of the invention is preferably carried out in the integrated plant of the invention which comprises an ethylbenzene dehydrogenator (1), a first work-up facility (2) for separating styrene and hydrogen from the reaction mixture produces in the ethylbenzene dehydrogenator (1), a hydrogen peroxide production facility (3), a propene epoxidizer (4) and a second work-up facility (5) for separating propene oxide from an epoxidation reaction mixture produced in the propene epoxidizer (4).

The ethylbenzene dehydrogenator (1) has an inlet for an ethylbenzene feed (15) and an outlet for produced reaction mixture and may be any device known from the prior art to be suitable for dehydrogenating ethylbenzene. In a preferred embodiment for adiabatic dehydrogenation, the ethylbenzene dehydrogenator comprises two adiabatic fixed bed reactors in a series each containing a fixed bed of a dehydrogenation catalyst and at least one heat exchanger between the two reactors for reheating the reaction mixture. In a preferred embodiment for isothermal dehydrogenation, the ethylbenzene dehydrogenator comprises a tube bundle reactor with a fixed bed of a dehydrogenation catalyst in the tubes of the tube bundle reactor, a shell enclosing the tube bundle and a heating medium circulation through the shell enclosing the tube bundle. Suitable reactors for dehydrogenating ethylbenzene are known from the prior art and are summarized in Ullmann's Encyclopedia of Industrial Chemistry Vol. 34, pages 532-534, DOI 10.1002/14356007.a25_329.pub2.

The first work-up facility (2) for separating styrene and hydrogen is connected to an outlet for reaction mixture of the ethylbenzene dehydrogenator (1) and comprises a hydrogen outlet and an outlet for separated styrene (17). The first work-up facility (2) preferably comprises a separation unit (16) for separating styrene and a crude hydrogen stream from the reaction mixture and a pressure swing adsorption unit (6). The hydrogen outlet of the first work-up facility (2) is then an outlet of the pressure swing adsorption unit (6) for carbon monoxide depleted hydrogen. The separation unit (16) preferably comprises a condenser (not shown in the figure) for condensing styrene, water, nonreacted ethylbenzene and organic by-products from the reaction mixture of the ethylbenzene dehydrogenator (1). The condenser has an outlet for non-condensed gas which provides the crude hydrogen stream. The separation unit (16) preferably further comprises a phase separator for separating the condensate of the condenser into an aqueous phase and an organic phase, a first distillation column for separating low boiling organic by-products from the organic phase as an overhead product, a second distillation column for separating ethylbenzene as an overhead product from the bottoms product of the first distillation column, and a third distillation column for separating a purified styrene from the bottoms product of the second distillation column.

The hydrogen peroxide production facility (3) has a hydrogen inlet, which is connected to a hydrogen outlet of the first work-up facility (2) and is preferably connected to an outlet of the pressure swing adsorption unit (6) for carbon monoxide depleted hydrogen, as well as an outlet for a hydrogen peroxide solution. The hydrogen peroxide production facility (3) is preferably a facility for producing hydrogen peroxide by an anthraquinone process and contains a working solution containing an alkylanthraquinone, an alkyltetrahydroanthraquinone or both. In this preferred embodiment, the hydrogen peroxide production facility (3) comprises a hydrogenator (7) for hydrogenating a working solution with hydrogen, an oxidizer (8) for oxidizing hydrogenated working solution with an oxygen containing gas, and an extraction column (9) for extracting hydrogen peroxide from oxidized working solution and an outlet for an aqueous hydrogen peroxide solution.

The hydrogenator (7) of the hydrogen peroxide production facility (3) is configured for hydrogenating a working solution which contains an alkylanthraquinone, an alkyltetrahydroanthraquinone or both, with hydrogen. For this purpose, the hydrogenator (7) has an inlet for extracted working solution and the hydrogen inlet of the hydrogen peroxide production facility (3), as well as an outlet for hydrogenated working solution. The hydrogenator (7) may be of any type known from the prior art for hydrogenating a working solution comprising an alkylanthraquinone, an alkyltetrahydroanthraquinone or both. The hydrogenator (7) may comprise a bubble column reactor, a stirred-tank reactor, a tube reactor, a fixed-bed reactor, a loop reactor or a gas-lift reactor for carrying out the hydrogenation reaction, depending on whether a suspended hydrogenation catalyst or a fixed bed hydrogenation catalyst shall be used. The hydrogenator (7) preferably comprises a bubble column with a recycle and injection of hydrogen gas at the column bottom for use with a suspended catalyst as known from WO 2010/139728 and Ullmann's Encyclopedia of Industrial Chemistry, online edition, entry "Hydrogen Peroxide", DOI: 10.1002/14356007.a13_443.pub3, pages 13-14 and Fig. 8. The hydrogenator (7) preferably comprises a heat exchanger for removing the heat of reaction from the working solution, preferably a heat exchanger arranged inside the hydrogenation reactor. When a suspended hydrogenation catalyst shall be used, the hydrogenator (7) typically also comprises a separator for separating catalyst from the working solution and returning it to the hydrogenation reactor, such as a filter, which may operate by cross-flow filtration or dead-end filtration. The hydrogenator (7) may also comprise a hydrogen compressor for carrying out hydrogenation at a pressure higher than the pressure provided at the hydrogen inlet of the hydrogen peroxide production facility (3). The hydrogenator (7) may further comprise a separator for separating non-reacted hydrogen gas from the hydrogenated working solution and recycling it to the hydrogenation reactor. If such a separator is present, the hydrogenator (7) preferably also comprises a recycle compressor for recycling the non-reacted hydrogen gas.

The oxidizer (8) of the hydrogen peroxide production facility (3) is configured for oxidizing hydrogenated working solution with an oxygen containing gas. For this purpose, the oxidizer (8) has an inlet for hydrogenated working solution connected to the outlet for hydrogenated working solution of the hydrogenator (7). The oxidizer (8) also has an inlet for an oxygen containing gas (18) as well as an outlet for off-gas (19) and an outlet for oxidized working solution. The oxidizer (8) comprises an oxidation reactor which may be of any type known from the prior art for oxidizing a hydrogenated working solution comprising an alkylanthrahydroquinone, an alkyltetrahydroanthrahydroquinone or both. Preferably, a bubble column, which is preferably operated in counter-current, is used as oxidation reactor. The bubble column can be free from internal devices, but preferably contains distribution devices in the form of packings or sieve plates, most preferably sieve plates in combination with internal heat exchangers. The oxidizer (8) preferably comprises a demister for separating droplets entrained in the off-gas leaving the oxidation reactor. The oxidizer (8) may further comprise a unit for recovering mechanical energy from off-gas leaving the oxidation reactor, such as a turboexpander as described in US 4,485,084 or a gas jet pump as described in WO 03/070632.

The extraction column (9) of the hydrogen peroxide production facility (3) is configured for extracting hydrogen peroxide from oxidized working solution. For this purpose, the extraction column (9) has an inlet for oxidized working solution connected to the outlet for oxidized working solution of the oxidizer (8), an inlet for an aqueous extractant (20), an outlet for an aqueous solution of hydrogen peroxide, and an outlet for extracted working solution. Any type of extraction column known from the prior art for extracting hydrogen peroxide with an aqueous extractant from oxidized working solution containing dissolved hydrogen peroxide may be used. The extraction column (9) is preferably a counter-current continuous extraction column, sieve-plate columns being most preferred. The hydrogen peroxide production facility (3) may additionally comprise a hydrogen peroxide purification unit for purifying the extracted aqueous hydrogen peroxide solution by removing working solution components, preferably a unit for washing the aqueous hydrogen peroxide solution with a solvent.

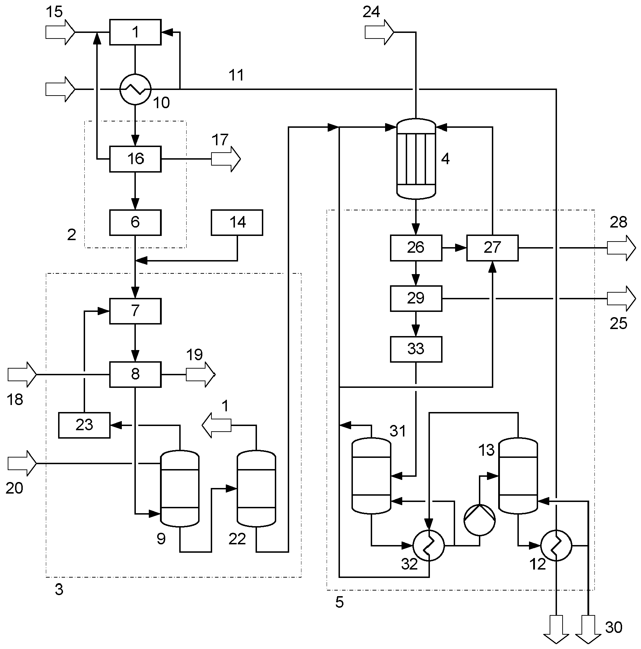

The hydrogen peroxide production facility (3) preferably also comprises a distillation unit configured for concentrating an aqueous hydrogen peroxide solution, which has an inlet for an aqueous solution of hydrogen peroxide, an outlet for a concentrated aqueous hydrogen peroxide solution and an outlet for separated water (21). The distillation unit typically comprises a hydrogen peroxide evaporator (not shown) and a distillation column (22) receiving vapor from the peroxide evaporator. Any type of hydrogen peroxide evaporator and distillation column known from the prior art for concentrating an aqueous hydrogen peroxide solution may be used. The hydrogen peroxide evaporator may be the distillation bottoms evaporator, which may be arranged separately from the distillation column (22) or may be integrated into the distillation column (22), for example as disclosed in EP 0 419 406 A1, Fig. 4 or in EP 0 835 680 A1, Fig. 1 and 2. A separate thermosiphon evaporator passing a two-phase mixture of vapor and liquid to the distillation column may be used as distillation bottoms evaporator. The distillation unit may also comprise both a hydrogen peroxide feed evaporator and a distillation bottoms evaporator, with compressed vapor being passed to the hydrogen peroxide feed evaporator, for example as disclosed in Fig. 1 and 2 of WO 2012/025333, or to the distillation bottoms evaporator or to both the hydrogen peroxide feed evaporator and the distillation bottoms evaporator. The distillation column (22) may comprise trays or packings or a combination of both and preferably comprises structured packings to minimize pressure drop in the column. The distillation unit may also comprise a vapor compressor receiving overhead vapor from the distillation column (22) and passing compressed vapor as heating medium to the hydrogen peroxide evaporator. The vapor compressor may be a mechanical compressor, preferably a one stage mechanical compressor and is most preferably a water ring pump. The vapor compressor may alternatively be a gas jet pump and is preferably a steam driven ejector.

The hydrogen peroxide production facility (3) preferably further comprises a drier (23) for reducing the water content of the extracted working solution before recycling it to the hydrogenator (7). Any type of drier known from the prior art to be suitable for removing water from the working solution of an anthraquinone process may be used.

The hydrogen peroxide production facility (3) may also comprises at least one buffer tank (not shown) for storing aqueous hydrogen peroxide solution produced by the hydrogen peroxide production facility (3).

The propene epoxidizer (4) is configured for epoxidizing propene with hydrogen peroxide to produce propene oxide as a product. For this purpose, the propene epoxidizer (4) comprises an epoxidation catalyst and has an inlet for a propene feed (24) and an inlet for hydrogen peroxide connected to the outlet for a hydrogen peroxide solution of the hydrogen peroxide production facility (3). The propene epoxidizer (4) preferably also has an inlet for a solvent used in the epoxidation reaction.

Any reactor known from the prior art to be useful for epoxidizing propene with hydrogen peroxide may be used as propene epoxidizer (4). When the epoxidation catalyst is a homogeneous catalyst, i.e. dissolved in the reaction mixture, or a suspended heterogeneous catalyst, the propene epoxidizer (4) preferably comprises a stirred tank reactor or a loop reactor. Preferably, the propene epoxidizer (4) comprises a fixed bed reactor which is configured for passing a mixture comprising propene, hydrogen peroxide and methanol over a fixed bed comprising a shaped titanium zeolite catalyst, preferably for passing the mixture over the catalyst fixed bed in downflow in a trickle bed mode. Preferably, a tube bundle reactor is used comprising fixed beds of the epoxidation catalyst within the tubes of the bundle. Such a tube bundle reactor preferably comprises from 5000 to 20000 parallel reaction tubes. The tube bundle reactor preferably has vertically arranged reaction tubes and comprises at least one distributor arranged above the entry of the reaction tubes, having openings for supplying liquid to each of the reaction tubes. The distributor preferably comprises separate openings for separately supplying two liquids to each of the reaction tubes, in particular for separately supplying a propene feed stream and a hydrogen peroxide feed stream to each of the reaction tubes. Suitable distributors are known from the prior art, for example from WO 2005/025716. This embodiment of the reactor is suitable for operating the process of the invention with trickle flow of liquid in the catalyst fixed bed. The propene epoxidizer (4) is preferably equipped with cooling means for cooling with a liquid cooling medium. Preferably, propene epoxidizer (4) comprises a tube bundle reactor with a cooling jacket enclosing the reaction tubes. The cooling jacket preferably has a feed point for cooling medium near the entry of the reaction tubes, a withdrawal point for cooling medium near the end of the reaction tubes and at least one additional withdrawal point for cooling medium upstream of the withdrawal point for cooling medium near the end of the reaction tubes. Preferably, a tube bundle reactor as described in WO 2017/089076 is used.

The second work-up facility (5) is configured for separating propene oxide and optionally solvent from an epoxidation reaction mixture produced in the propene epoxidizer (4) and comprises an inlet for reaction mixture connected to the outlet for reaction mixture of the propene epoxidizer (4) as well as an outlet for separated propene oxide (25) for this purpose. The second work-up facility (5) preferably comprises units for separating and recovering non-reacted propene from the epoxidation reaction mixture, units for separating and purifying propene oxide and a unit for separating solvent used in the epoxidation reaction.

The propene separation unit (26) for separating non-reacted propene from the epoxidation reaction mixture preferably comprises a pressure reduction unit having the inlet for reaction mixture, an outlet for propene rich vapor and an outlet for propene depleted reaction mixture. The pressure reduction unit preferably comprises a flash evaporator and more preferably a series of flash evaporators and corresponding compressors as described in WO 2017/089079 on page 4, line 32 to page 6, line 3.

The propene recovery unit (27) preferably comprises a condenser connected to the outlet for propene rich vapor of the propene separation unit (26), an outlet for recovered liquid propene and an outlet for off-gas (28). The propene recovery unit (27) preferably comprises a propene rectifier column for separating the vapor provided by flash evaporators of the propene separation unit (26) into a liquid overhead propene stream, a bottoms stream containing higher boiling components and an overhead gas stream containing propene, oxygen and nitrogen added for inertisation. The propene rectifier is preferably also connected to the propene epoxidizer (4)) for receiving an off-gas stream. The propene recovery unit (27) preferably also comprises an absorption unit for absorbing propene from non-condensed gas into a solvent, preferably solvent recovered from the reaction mixture.

The propene oxide separation unit (29) has an inlet for propene depleted reaction mixture connected to the outlet for propene depleted reaction mixture of the propene separation unit (26) and the outlet for separated propene oxide (25). The propene oxide separation unit (29) preferably comprises a pre-separation column with the inlet for propene depleted reaction mixture, which preferably has from 5 to 20 theoretical separation stages in the stripping section and less than 3 theoretical separation stages in a rectifying section. If the propene recovery unit (27) comprises a propene rectifier column, the pre-separation column also receives the bottoms product of the propene rectifier column. The propene oxide separation unit (29) preferably further comprises a propene stripper column receiving the overhead product of the pre-separation column and providing an overhead stream enriched in propene which, if a propene rectifier column is used, is passed to the propene rectifier column. The propene depleted bottoms product from the propene stripper column is preferably passed to a propene oxide purification column which is preferably configured for purifying the feed by an extractive distillation, preferably an extractive distillation with addition of a compound containing an NH₂ group reactive to acetaldehyde as described in WO 2004/048355 and WO 2017/093204.

The unit for separating solvent has an inlet connected to an outlet of the propene oxide separation unit (29) for reaction mixture depleted in propene and propene oxide, an outlet for recovered solvent connected to an inlet for solvent of the propene epoxidizer (4), and an outlet for waste water (30). The unit for separating solvent preferably comprises at least one distillation column (13) with a heat exchanger (12) supplying heat to the distillation column (13). Preferably, the unit for separating the solvent comprises two heat integrated distillation columns (13, 31) in series, each with a bottoms evaporator (12, 32), configured for operating the second distillation column (13) at a higher pressure than the first distillation column (31) and for passing overhead vapor from the second distillation column (13) as heating medium to the bottoms evaporator (32) of the first distillation column (31). The unit for separating the solvent preferably receives the bottoms product from a pre-separation column as described above. If the propene oxide separation unit (29) comprises an extractive distillation, the unit for separating the solvent preferably also receives the bottoms product from the extractive distillation.

The second work-up facility (5) preferably also comprises a hydrogenation reactor (33) downstream of the propene oxide separation unit (29) and upstream of the unit for separating solvent. This hydrogenation reactor (33) preferably comprises a fixed bed containing a heterogeneous hydrogenation catalyst and is preferably configured for operation with downflow of liquid in a trickle flow mode. If the propene oxide separation unit (29) comprises a pre-separation column and an extractive distillation as described above, preferably the bottoms product from both these units is passed to the hydrogenation reactor (33).

In a preferred embodiment, the integrated plant of the invention further comprises a steam generator (10) heated by reaction mixture exiting the ethylbenzene dehydrogenator (1) and a conduit (11) connecting a steam outlet of the steam generator (10) with a heating medium inlet of a heat exchanger (12) of a distillation column (13) of the second work-up facility (5).

The integrated plant of the invention may also comprise an additional hydrogen generator (14) with a hydrogen outlet connected to the hydrogen inlet of the hydrogen peroxide production facility (3). The additional hydrogen generator (14) preferably comprises a steam reformer.

### List of reference signs:

- 1: Ethylbenzene dehydrogenator
- 2: First work-up facility
- 3: Hydrogen peroxide production facility
- 4: Propene epoxidizer
- 5: Second work-up facility
- 6: Pressure swing adsorption unit
- 7: Hydrogenator
- 8: Oxidizer
- 9: Extraction column
- 10: Steam generator
- 11: Conduit connecting steam generator (10) with heat exchanger (12)
- 12: Heat exchanger
- 13: First distillation column
- 14: Hydrogen generator
- 15: Ethylbenzene feed
- 16: Separation unit for separating styrene and a crude hydrogen stream
- 17: Separated styrene
- 18: Oxygen containing gas
- 19: Off-gas
- 20: Aqueous extractant
- 21: Separated water
- 22: Distillation column
- 23: Drier
- 24: Propene feed
- 25: Propene oxide
- 26: Propene separation unit
- 27: Propene recovery unit
- 28: Off-gas
- 29: Propene oxide separation unit
- 30: Waste water
- 31: First distillation column
- 32: Bottoms evaporator

## Claims

1. An integrated process for making styrene and propene oxide comprising the steps
a) dehydrogenating ethylbenzene in the presence of a dehydrogenation catalyst;
b) separating styrene and hydrogen from the reaction mixture of step a);
c) producing hydrogen peroxide from hydrogen separated in step b) and oxygen;
d) reacting propene with the hydrogen peroxide obtained in step c) in the presence of an epoxidation catalyst to provide a reaction mixture comprising propene oxide; and
e) separating propene oxide from the reaction mixture obtained in step d).

2. The integrated process of claim 1, wherein step a) is carried out in the presence of steam, step c) uses a palladium metal catalyst and step b) comprises removal of carbon monoxide from separated hydrogen.

3. The integrated process of claim 2, wherein carbon monoxide is removed by pressure swing adsorption.

4. The integrated process of any one of claims 1 to 3, wherein step c) comprises the steps
c1) hydrogenating a working solution, containing an alkylanthraquinone, an alkyltetrahydroanthraquinone or both, with hydrogen separated in step b) in a hydrogenation reactor in the presence of the palladium metal catalyst to provide a hydrogenated working solution comprising an alkylanthrahydroquinone, an alkyltetrahydroanthrahydroquinone or both;
c2) oxidizing hydrogenated working solution obtained in step c1) with an oxygen containing gas in an oxidation reactor to provide an oxidized working solution comprising hydrogen peroxide and an alkylanthraquinone, an alkyltetrahydroanthraquinone or both;
c3) extracting hydrogen peroxide from oxidized working solution obtained in step c2) to provide an aqueous solution of hydrogen peroxide;

5. The integrated process of any one of claims 1 to 4, wherein step b) comprises separating nonreacted ethylbenzene from the reaction mixture of step a) and recycling the separated ethylbenzene to step a).

6. The integrated process of any one of claims 1 to 5, wherein the epoxidation catalyst is a titanium zeolite catalyst and step d) is carried out in the presence of a solvent.

7. The integrated process of claim 6, wherein step e) comprises separating the solvent from the reaction mixture obtained in step d), the reaction mixture of step a) is passed through a steam generator prior to separation in step b) and steam generated in the steam generator is used in step e) to provide heat for separating the solvent.

8. The integrated process of any one of claims 1 to 7, comprising an additional step of generating hydrogen which is supplied to step c).

9. An integrated plant for making styrene and propene oxide comprising
a) an ethylbenzene dehydrogenator (1) comprising a dehydrogenation catalyst;
b) a first work-up facility (2) for separating styrene and hydrogen connected to an outlet for reaction mixture of the ethylbenzene dehydrogenator (1);
c) a hydrogen peroxide production facility (3) with a hydrogen inlet connected to a hydrogen outlet of the first work-up facility (2);
d) a propene epoxidizer (4) connected to a hydrogen peroxide outlet of the hydrogen peroxide production facility (3) and comprising an epoxidation catalyst; and
e) a second work-up facility (5) for separating propene oxide from an epoxidation reaction mixture connected to an outlet for reaction mixture of the propene epoxidizer (4).

10. The integrated plant of claim 9, wherein the first work-up facility (2) comprises a pressure swing adsorption unit (6) and the hydrogen outlet of the first work-up facility (2) is an outlet of the pressure swing adsorption unit (6) for carbon monoxide depleted hydrogen.

11. The integrated plant of claim 9 or 10, wherein the hydrogen peroxide production facility comprises
c1) a hydrogenator (7) for hydrogenating a working solution, containing an alkylanthraquinone, an alkyltetrahydroanthraquinone or both, with hydrogen;
c2) an oxidizer (8) for oxidizing hydrogenated working solution with an oxygen containing gas; and
c3) an extraction column (9) for extracting hydrogen peroxide from oxidized working solution.

12. The integrated plant of any one of claims 9 to 11, comprising a steam generator (10) heated by reaction mixture exiting the ethylbenzene dehydrogenator(1) and a conduit (11) connecting a steam outlet of the steam generator (10) with a heating medium inlet of a heat exchanger (12) of a distillation column (13) of the second work-up facility (5).

13. The integrated plant of claim 9 to 12, comprising an additional hydrogen generator (14) with a hydrogen outlet connected to the hydrogen inlet of the hydrogen peroxide production facility (3).

14. The integrated plant of claim 13, wherein the additional hydrogen generator (14) comprises a steam reformer.
